(19) **Europäisches Patentamt / European Patent Office / Office européen des brevets**

(11) **EP 2 301 499 B1**

(12) **EUROPEAN PATENT SPECIFICATION**

(45) Date of publication and mention
of the grant of the patent:
**06.04.2022 Bulletin 2022/14**

(21) Application number: **09794341.9**

(22) Date of filing: **29.06.2009**

(51) International Patent Classification (IPC):
*A61F 13/15* (2006.01)     *A61F 13/53* (2006.01)
*A61F 13/534* (2006.01)     *B32B 27/12* (2006.01)
*A61F 13/539* (2006.01)     *B32B 5/02* (2006.01)
*B32B 5/26* (2006.01)     *B32B 7/12* (2006.01)

(52) Cooperative Patent Classification (CPC):
**A61F 13/15203; A61F 13/53; A61F 13/534;
A61F 13/539; B32B 5/022; B32B 5/26; B32B 7/12;**
A61F 2013/530299; A61F 2013/530481;
A61F 2013/530554; A61F 2013/5307;
A61F 2013/53445; A61F 2013/53908;
A61F 2013/53958; B32B 2250/20;     (Cont.)

(86) International application number:
**PCT/JP2009/061813**

(87) International publication number:
**WO 2010/004895 (14.01.2010 Gazette 2010/02)**

(54) **WATER ABSORBING SHEET COMPOSITE**

WASSERABSORBIERENDER BAHNVERBUND

COMPOSITION DE FEUILLE HYDRO-ABSORBANTE

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **11.07.2008 JP 2008182029**

(43) Date of publication of application:
**30.03.2011 Bulletin 2011/13**

(73) Proprietor: **SUMITOMO SEIKA CHEMICALS CO.,
LTD.
Kako-gun, Hyogo 675-0145 (JP)**

(72) Inventors:
• **TAKATORI, Junichi
Hyogo 672-8076 (JP)**
• **HANDA, Masayoshi
Hyogo 672-8076 (JP)**
• **FUKUDOME, Shinya
Hyogo 672-8076 (JP)**
• **MAEDA, Nobuhiro
Hyogo 672-8076 (JP)**

(74) Representative: **Hoffmann Eitle
Patent- und Rechtsanwälte PartmbB
Arabellastraße 30
81925 München (DE)**

(56) References cited:
EP-A1- 0 875 225     EP-A1- 2 383 115
EP-A1- 2 387 981     WO-A1-00/38749
WO-A1-03/063746     GB-A- 2 269 109
JP-A- 2001 252 307     JP-A- 2004 313 580
JP-B2- 2 963 647     JP-B2- 63 003 062
JP-U- 6 058 931     JP-U- 6 058 952
JP-U- 6 059 039     US-A- 5 821 179
US-A1- 2006 009 743     US-A1- 2007 219 518

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(52) Cooperative Patent Classification (CPC): (Cont.)
   B32B 2250/40; B32B 2255/02; B32B 2255/26;
   B32B 2264/02; B32B 2264/12; B32B 2307/728;
   B32B 2555/02

**Description**

WATER-ABSORBENT SHEET COMPOSITION

TECHNICAL FIELD

The present invention relates to a water-absorbent sheet composition which can be used in the field of hygienic materials. More specifically, the present invention relates to a water-absorbent sheet composition which can be suitably used in absorbent articles, such as thin-style sanitary napkins and incontinence pads.

BACKGROUND ART

Body liquid absorbent articles for use in hygienic materials such as sanitary napkins have a structure in which an absorbent material for absorbing a body liquid such as menstrual blood or urine is sandwiched between a flexible liquid-permeable surface sheet (top sheet) positioned on a side contacting a body and a liquid-impermeable backside sheet (back sheet) positioned on a side opposite to that contacting the body.
In recent years, there are increasing demands on thinning and lighter weights of hygienic materials such as sanitary napkins, from alleviating discomfort upon fitting or convenience of transportation. In addition, there are also increasing demands on the prevention of the absorbent material from liquid leakage after the absorption. On the other hand, a method for thinning that is generally performed in a conventional hygienic material is, for example, a method of reducing a hydrophilic fiber such as pulp fiber, which serves to fix a water-absorbent resin in an absorbent material, and increasing the water-absorbent resin.
As described above, an absorbent material in which a water-absorbent resin is used in a large amount with a lowered proportion of a hydrophilic fiber is preferred in thinning, from the viewpoint of reducing bulky hydrophilic fibers while retaining a larger amount of a liquid and being capable of preventing liquid leakage. However, when a liquid distribution or diffusion upon actually using in a hygienic material such as sanitary napkins and incontinence pads is considered, there is a disadvantage that if a large amount of the water-absorbent resin is formed into a soft gel-like state by absorption, a so-called "gel-blocking phenomenon" takes place, whereby liquid diffusibility is markedly lowered and a liquid permeation rate of the absorbent material is slowed down. This "gel-blocking phenomenon" is a phenomenon in which especially when an absorbent material in which water-absorbent resins are highly densified absorbs a liquid, a water-absorbent resin existing near a surface layer absorbs the liquid to even more densify soft gel that forms near the surface layer, so that a liquid permeation into an internal of an absorbent material is inhibited, thereby making the internal of the water-absorbent resin incapable of efficiently absorbing the liquid. Further, an absorbent material having reduced hydrophilic fibers that contributes to shape retention is likely to undergo deformation of shape, due to twisting or tear before or after the absorption of a liquid, The absorbent material that undergoes deformation of shape has markedly lowered liquid diffusibility, so that the permeation rate is slowed down and a liquid leakage occurs, without being able to exhibit the ability of the absorbent material. In order to avoid such phenomena and maintain the absorption properties of the absorbent material, a ratio of hydrophilic fibers and a water-absorbent resin is limited, thereby posing limitations on the reduction of hydrophilic fibers even to the thinning of hygienic materials.
In view of the above, conventionally, as a means of inhibiting "gel blocking phenomenon" which occurs when reducing hydrophilic fibers and using a water-absorbent resin in a large amount, for example, proposals such as a method using two kinds of water-absorbent resins having different water-absorbent capabilities (see Patent Publication 1) and a method using a water-absorbent resin having a high surface cross-linking density (see Patent Publication 2) have been made.
However, in these methods, the absorption properties as the absorbent materials in which water-absorbent resins are used in large amounts cannot be satisfied. In addition, there arise some problems that the water-absorbent resin is localized before use and subject to be mobile during use because hydrophilic fibers that play a role of fixing the water-absorbent resin are reduced, so that the "gel-blocking phenomenon" is more likely to take place.
In order to solve these problems, a method of immobilizing a water-absorbent resin to hydrophilic fibers has been studied. For example, proposals such as a method of allowing thermoplastic adhesive fibers to be contained in an absorbent material comprising a water-absorbent resin and a hydrophilic fiber, and heat-fusing the absorbent material (see Patent Publication 3), a method of immobilizing a water-absorbent resin and hydrophilic fibers with an emulsion adhesive, and a method of immobilizing a water-absorbent resin to a substrate with a hot melt adhesive (see Patent Publication 4) have been made.
However, when a water-absorbent resin is immobilized with an adhesive according to the method as described above, the swelling of the water-absorbent resin is likely to be suppressed because the water-absorbent resin is bound to the adhesive. Especially when a water-absorbent resin and hydrophilic fibers are immobilized with a thermoplastic adhesive or an emulsion, there arise some problems that the water absorbency inherently owned by the water-absorbent resin would not be sufficiently exhibited, and the "gel-blocking phenomenon" is likely to take place.

There is also a method of immobilizing a water-absorbent resin to a substrate without using an adhesive, which is, for example, a method of adhering water-absorbent polymer particles in the process of polymerization to a fibrous substrate to carry out polymerization on the fibrous substrate (see Patent Publication 5) and a method of polymerizing a monomer aqueous composition containing acrylic acid and an acrylic acid salt as main components on a nonwoven fabric substrate by means of electron beam irradiation (see Patent Publication 6).

In these methods, while the fibrous substrate is penetrated into the polymer particles to be firmly adhered, there are some disadvantages that it is difficult to complete the polymerization reaction in the substrate, so that unreacted monomers remain in the substrate in large amounts.

US 2007/219518 A1 describes an absorbent article including a cover layer, a barrier layer and an absorbent system arranged between the cover layer and the barrier layer. According to an example, a sanitary napkin comprises spunlace cover layer (75 g/m$^2$) and a 23 $\mu$m (0.9 mil) polyethylene film as a barrier layer. In between the cover layer and the barrier layer, glue and absorbent filler, a tissue paper, and glue are arranged in this order.

In WO 03/063746 A1, an absorbent article having a first essentially liquid permeable surface layer and an essentially liquid impermeable backing layer is described. An absorbent body is located in between.

US 2006/009743 A1 is concerned with an absorbent article which includes a stretchable substrate layer and an absorbent core formed directly on the substrate layer, and adhesively attached to the substrate layer. The absorbent core includes about 40-99% by weight superabsorbent particles and about 1-60% by weight adhesive fibers.

US 5,821,179 describes an absorbent sheet comprising at least hydrophilic fibers and thermally fusible bonding fibers or a strengthening assistant, and a superabsorbent polymer. The superabsorbent polymer is not present on an absorbent surface of the absorbent sheet for absorbing liquid but distributed inside the absorbent sheet, and is adhered and fixed to the hydrophilic fibers constituting the absorbent sheet.

JP 2004-313580 A is concerned with the disposable absorbent article using an absorbent body free from pulp fiber other than a cloth-like nonwoven. This absorbent body comprises a laminated sheet body made by laminating two or more layers of an absorbent sheet body made by sandwiching and bonding an absorbent resin powder between a first nonwoven cloth layer and a second nonwoven cloth layer such that there are absorbent resin powder existing areas and absorbent resin powder absent areas.

In WO 00/38749 A1, a composite absorbent structure is described, which comprises: a) a first wicking layer comprising wettable fibers; b) a second retention layer comprising a hydrogel-forming polymeric material; and c) a bonding agent for bonding said first wicking layer and said second retention layer to form a composite absorbent structure. The bonding agent may comprise Danaklon fibers.

Further prior art water-absorbent sheet compositions are disclosed in GB 2 269 109 A, EP 0 875 225 A1 and JP 2 963647 B2.

## PRIOR ART PUBLICATIONS

### PATENT PUBLICATIONS

    Patent Publication 1: Japanese Patent Laid-Open No. 2001-252307
    Patent Publication 2: Japanese Patent Laid-Open No. Hei-06-057010
    Patent Publication 3: Japanese Patent Laid-Open No. Hei-10-118114
    Patent Publication 4: Japanese Patent Laid-Open No. 2000-238161
    Patent Publication 5: Japanese Patent Laid-Open No. 2003-11118
    Patent Publication 6: Japanese Patent Laid-Open No. Hei-02-048944

## DISCLOSURE OF INVENTION

## PROBLEMS TO BE SOLVED BY THE INVENTION

An object of the present invention is to provide a water-absorbent sheet composition which has sufficient absorbency without undergoing deformation of shape after liquid absorption, while being thin in style, so that the sheet composition can be suitably used as sanitary napkins and incontinence pads.

## MEANS TO SOLVE THE PROBLEMS

The present invention relates to:

a water-absorbent sheet composition as defined by claim 1. Further embodiments are defined by dependent claims 2 and 3.

EFFECTS OF THE INVENTION

The water-absorbent sheet composition of the present invention exhibits some excellent effects that the sheet composition has sufficient absorbency not only for a liquid having a low viscosity but also a liquid having a high viscosity, without undergoing deformation of shape before or after liquid absorption, while being thin in style. Therefore, the water-absorbent sheet composition of the present invention is used as an absorbent material for sanitary napkins and incontinence pads, whereby a hygienic material not only that is thin and has excellent external design, but also free from inconveniences such as liquid leakage can be provided. In addition, the water-absorbent sheet composition of the present invention can be used in the field of agriculture and the field of construction materials in addition to the field of hygienic materials.

BRIEF DESCRIPTION OF THE DRAWINGS

[Figure 1] A schematic view when a test liquid is supplied, in the property evaluation of the water-absorbent sheet composition.
[Figure 2] A schematic view when an amount of re-wet is measured, in the property evaluation of the water-absorbent sheet composition.
[Figure 3] A schematic view when diffusion length is measured, in the property evaluation of the water-absorbent sheet composition.

BEST MODE FOR CARRYING OUT THE INVENTION

The water-absorbent sheet composition has a structure in which a water-absorbent resin and a hot melt adhesive are sandwiched with two or more sheets of hydrophilic nonwoven fabrics, and a thin-style water-absorbent sheet composition that does not contain hydrophilic fibers such as pulp fibers between the hydrophilic nonwoven fabrics can be realized by adjusting an amount of the water-absorbent resin and an amount of the hot melt adhesive based on the water-absorbent resin. Further, in the water-absorbent sheet composition of the present invention, since a water-absorbent resin is immobilized to a nonwoven fabric with a hot melt adhesive, the localization or scattering of the water-absorbent resin can be inhibited, even though the sheet composition does not contain hydrophilic fibers such as pulp fibers between the hydrophilic nonwoven fabrics, and the deformation of shape is also inhibited. In addition, an entire surface of the water-absorbent resin is not in the state that is covered with a hot melt adhesive, but a part is in the state of being immobilized; therefore, it is considered that the water-absorbent resin can be sufficiently swollen, without inhibiting water absorbency of the water-absorbent resin.
From the viewpoint of thinning, the water-absorbent sheet composition of the present invention does not contain hydrophilic fibers such as pulp fibers admixed with the water-absorbent resin between the nonwoven fabrics.
As the kinds of the water-absorbent resins, commercially available water-absorbent resins can be used. For example, the water absorbent resin includes hydrolysates of starch-acrylonitrile graft copolymers, neutralized products of starch-acrylic acid graft polymers, saponified products of vinyl acetate-acrylic acid ester copolymers, and partially neutralized products of polyacrylic acid. Among them, the partially neutralized products of polyacrylic acids are preferred, from the viewpoint of amount of production, production costs, and water absorbency.
The partially neutralized product of a polyacrylic acid has a degree of neutralization of preferably 50% by mol or more, and even more preferably from 70 to 90% by mol, from the viewpoint of increasing osmotic pressure of the water-absorbent resin, thereby increasing water absorbency.
The water-absorbent resin is contained in an amount of from 30 to 100 $g/m^2$ from the viewpoint of obtaining sufficient absorbency even when the water-absorbent sheet composition of the present invention is used as sanitary napkins or incontinence pads. When the water-absorbent resin is contained in an amount of less than 10 $g/m^2$, sufficient absorbency cannot be obtained as an absorbent material, so that an amount of re-wet is likely to increase. When the water-absorbent resin is contained in an amount exceeding 150 $g/m^2$, the gel-blocking phenomenon is more likely to take place, so that diffusion property of the liquid is worsened as an absorbent material, and the permeation rate is likely to be slowed down.
The absorbency of the water-absorbent sheet composition of the present invention is influenced by the water absorbency of the water-absorbent resin used. Therefore, it is preferable that the water-absorbent resin to be used in the present invention is those selected with optimal ranges in water absorption properties such as water absorption capacity (water-retention capacity), water absorption capacity under load, and water absorption rate of the water-absorbent resin, by taking the constitution of each component of the water-absorbent sheet composition into consideration.

The water-absorbent resin has a water-retention capacity of physiological saline solution of from 35 to 65 g/g, preferably from 40 to 60 g/g, from the viewpoint of absorbing a liquid in a larger amount, and preventing the gel blocking phenomenon while keeping the gel strong during absorption. The water-retention capacity of physiological saline solution of the water-absorbent resin is a value obtainable by a measurement method described in Examples set forth below.

The water-absorbent resin has a water-absorption rate of physiological saline solution of less than 40 s, preferably from 1 to 30 s, from the viewpoint of speeding up the permeation rate of the water-absorbent sheet composition of the present invention and preventing a liquid leakage upon use in a hygienic material. The water-absorption rate of the water-absorbent resin is a value obtainable by a measurement method described in Examples set forth below.

The water-absorbent resin has a mass-average particle size of from 60 to 500 pm, and preferably from 100 to 400 pm, from the viewpoint of preventing the scattering of the water-absorbent resin, the gel blocking phenomenon during water absorption and water absorption of the water-absorbent resin in the water-absorbent sheet composition, and at the same time reducing the rugged feel of the water-absorbent sheet composition, thereby improving texture.

The hot melt adhesive used in the present invention includes, for example, rubber adhesives such as natural rubbers, butyl rubbers, and polyisoprene; styrenic elastomer adhesives such as styrene-isoprene block copolymers (SIS), styrene-butadiene block copolymers (SBS), styrene-isobutylene block copolymers (SIBS), and styrene-ethylene-butylene-styrene block copolymers (SEBS); ethylene-vinyl acetate copolymer (EVA) adhesives; ethylene-acrylic acid derivative copolymer adhesives such as ethylene-ethyl acrylate copolymer (EEA), and ethylene-butyl acrylate copolymer (EBA); ethylene-acrylic acid copolymer (EAA) adhesives; polyamide adhesives such as copolymer nylons and dimer acids-based polyamides; polyolefin adhesives such as polyethylenes, polypropylenes, atactic polypropylenes, and copolymeric polyolefins; polyester adhesives such as polyethylene terephthalate (PET), polybutylene terephthalate (PBT), and copolymeric polyesters; and acrylic adhesives, and these adhesives may be used together in two or more kinds. In the present invention, the ethylene-vinyl acetate copolymer adhesives and the styrenic elastomer adhesives are preferred, from the viewpoint of high adhesive strength, thereby making it possible to prevent exfoliation of a hydrophilic nonwoven fabric and scattering of the water-absorbent resin.

The hot melt adhesive has a melting temperature or a softening point of preferably from 60° to 180°C, from the viewpoint of sufficiently fixing a water-absorbent resin to a nonwoven fabric and preventing thermal deterioration or deformation of the nonwoven fabric. Here, in the water-absorbent sheet composition of the present invention, in the process of producing a water-absorbent sheet composition, after melting, the hot melt adhesive is adhered to a nonwoven fabric or a water-absorbent resin in a solid state by cooling the molten adhesive.

The hot melt adhesive is contained in an amount of from 0.20 to 0.5 times the amount of the water-absorbent resin contained (mass basis). When the hot melt adhesive is contained in an amount of less than 0.10 times, adhesion becomes insufficient, thereby making likely to cause exfoliation of the hydrophilic nonwoven fabrics themselves and increase in localization or scattering of the water-absorbent resin. When the hot melt adhesive is contained in an amount exceeding 1.0 time, the adhesion of the hydrophilic nonwoven fabrics is too strong to each other, and the swelling of the water-absorbent resin after water absorption is inhibited, thereby making it likely to increase amount of re-wet.

The water-absorbent sheet composition of the present invention also has a feature in the aspect in the use of two or more hydrophilic nonwoven fabrics. In general, when a water-absorbent sheet composition is used in a hygienic material, it is deduced that properties as the hygienic material such as permeation rate or amount of re-wet are greatly influenced by various properties such as hydrophilicity or strength of a nonwoven fabric on a liquid-permeable side (front side) in the water-absorbent sheet composition. In addition, in a case of a nonwoven fabric on a reverse side, it is deduced that the properties other than strength or flexibility are not as necessary as in the front side. However, during the present study, when only a nonwoven fabric of a reverse side is made hydrophobic, although the reasons therefor are not clear, a significantly large amount of liquid leakage is caused between the nonwoven fabrics during water absorption, so that it is found that a nonwoven fabric on a reverse side is also obliged to be hydrophilic. In other words, in the present invention, a thin-style water-absorbent sheet composition having high absorbency can be obtained by using hydrophilic nonwoven fabrics on both the sides of the nonwoven fabrics sandwiching the water-absorbent resin.

The hydrophilic nonwoven fabric used in the present invention is not particularly limited, so long as the hydrophilic nonwoven fabric is a known nonwoven fabric in the field of art. The hydrophilic nonwoven fabric includes nonwoven fabrics made of polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fibers, and other synthetic fibers; nonwoven fabrics produced by mixing, for example, cotton, silk, flax or pulp (cellulose) fibers from the viewpoint of liquid permeability, flexibility and strength upon forming into a sheet composition, and the hydrophilic nonwoven fabric may be a mixture of two or more kinds of fibers. In addition, its surface may be subjected to a hydrophilic treatment according to a known method, as occasion demands. The nonwoven fabric made of synthetic fibers is preferably used, from the viewpoint of increasing the strength of the water-absorbent sheet composition, and especially at least one member selected from the group consisting of rayon fibers, polyolefin fibers, polyester fibers, and mixtures thereof is preferred. The hydrophilic nonwoven fabric made of synthetic fibers may contain pulp fibers in a small amount to an extent that the thickness of the water-absorbent sheet composition would not increase.

The hydrophilic nonwoven fabric is preferably a nonwoven fabric having an appropriate bulkiness and a large basis weight, from the viewpoint of giving the water-absorbent sheet composition of the present invention excellent liquid permeability, flexibility, strength and cushioning property, and speeding up the permeation rate of the water-absorbent sheet composition. The hydrophilic nonwoven fabric has a basis weight of from 50 to 150 $g/m^2$.

The water-absorbent sheet composition of the present invention can be produced, for example, by uniformly dispersing mixed powders of a water-absorbent resin and a hot melt adhesive on a hydrophilic nonwoven fabric, further overlaying a hydrophilic nonwoven fabric over the hydrophilic nonwoven fabric, and heat-and-pressing the overlaid fabrics at a temperature near a melting temperature of the hot melt adhesive.

In addition, the water-absorbent sheet composition of the present invention may properly be formulated with an additive such as a deodorant, a bactericidal agent, or a gel stabilizer.

The water-absorbent sheet composition of the present invention has a great feature in the aspect of enabling thinning of the composition. When the use in sanitary napkins or incontinence pads is taken into consideration, the water-absorbent sheet has a thickness, on a dry basis, of preferably 4 mm or less, more preferably 3 mm or less, even more preferably 2 mm or less, and still even more preferably from 0.5 to 2 mm.

Further, an absorbent article may have a structure in which a water-absorbent sheet composition of the present invention is sandwiched between a liquid-permeable sheet and a liquid-impermeable sheet. Such an absorbent article includes, for example, incontinence pads, sanitary napkins, pet sheets, drip sheets for foods, and water blocking materials for electric power cables. Further, as the liquid-permeable sheet and the liquid-impermeable sheet, known ones in the technical field of the absorbent articles can be used without particular limitations. The absorbent article can be produced by a known method.

EXAMPLES

The present invention will be specifically described hereinbelow by the Examples, without intending to limit the scope of the present invention thereto.

The properties of the water-absorbent resin and the water-absorbent sheet composition were measured in accordance with the following methods.

< Water-Retention Capacity of Saline Solution of Water-Absorbent Resin >

The amount 2.0 g of water-absorbent resin was weighed in a cotton bag (Cottonbroad No. 60, width 100 mm × length 200 mm), and placed in a 500 mL-beaker. Physiological saline (0.9% by mass aqueous solution of sodium chloride, hereinafter referred to the same) was poured into the cotton bag in an amount of 500 g at one time, and the physiological saline was dispersed so as not to generate an unswollen lump of the water-absorbent resin. The upper part of the cotton bag was tied up with a rubber band, and the cotton bag was allowed to stand for 1 hour, to sufficiently swell the water-absorbent resin. The cotton bag was dehydrated for 1 minute with a dehydrator (manufactured by Kokusan Enshinki Co., Ltd., product number: H-122) set to have a centrifugal force of 167G. The mass Wa (g) of the cotton bag containing swollen gels after the dehydration was measured. The same procedures were carried out without adding water-absorbent resin, and the empty mass Wb (g) of the cotton bag upon wetting was measured. The water-retention capacity of saline solution of the water-absorbent resin was calculated from the following formula.

$$\text{Water-Retention Capacity of Saline Solution (g/g) of}$$

$$\text{Water-Absorbent Resin}$$

$$= [Wa - Wb] \, (g) / \text{Mass (g) of Water-Absorbent Resin}$$

< Water Absorption Rate of Saline Solution of Water-Absorbent Resin >

This test was conducted in a room temperature-controlled to 25° ± 1°C. The amount 50 ± 0.1 g of physiological saline was weighed out in a 100 mL beaker, and a magnetic stirrer bar (8 mm$\phi$ × 30 mm, without a ring) was placed therein. The beaker was immersed in a thermostat, of which liquid temperature was controlled to 25° ± 0.2°C. Next, the beaker was placed over the magnetic stirrer so that a vortex was generated in physiological saline at a rotational speed of 600 r/min, the water-absorbent resin was then quickly added in an amount of 2.0 ± 0.002 g to the above beaker, and the time period (seconds) from a point of addition of the water-absorbent resin was added to a point of convergence of the vortex of the liquid surface was measured with a stopwatch , which was defined as a water absorption rate of the water-absorbent resin.

< Mass-Average Particle Size of Water-Absorbent Resin >

An amorphous silica (Sipernat 200, Degussa Japan) was mixed in an amount of 0.5 g as a lubricant with 100 g of a water-absorbent resin.

The above-mentioned water-absorbent resin particles were allowed to pass though a JIS standard sieve having a sieve opening of 250 μm, and a mass-average particle size was measured using a combination of sieves of (A) in a case where the particles are allowed to pass in an amount of 50% by mass or more, or a combination of sieves of (B) in a case where 50% by mass or more of the particles remain on the sieve.

(A) JIS standard sieves, a sieve having an opening of 425 μm, a sieve having an opening of 250 μm, a sieve having an opening of 180 μm, a sieve having an opening of 150 μm, a sieve having an opening of 106 μm, a sieve having an opening of 75 μm, a sieve having an opening of 45 μm, and a receiving tray were combined in order from the top.
(B) JIS standard sieves, a sieve having an opening of 850 μm, a sieve having an opening of 600 μm, a sieve having an opening of 500 μm, a sieve having an opening of 425 μm, a sieve having an opening of 300 μm, a sieve having an opening of 250 μm, a sieve having an opening of 150 μm, and a receiving tray were combined in order from the top.

The above-mentioned water-absorbent resin particles were placed on an uppermost sieve of the combined sieves, and shaken for 20 minutes with a rotating and tapping shaker machine to classify the particles.

After classification, the relationships between the opening of the sieve and an integral of a mass percentage of the water-absorbent resin remaining on the sieve were plotted on a logarithmic probability paper by calculating the mass of the water-absorbent resin remaining on each sieve as a mass percentage to an entire amount, and accumulating the mass percentages in order, starting from those having larger particle diameters. A particle diameter corresponding to a 50% by mass cumulative mass percentage is defined as a mass-average particle size by joining the plots on the probability paper in a straight line.

< Evaluation of Properties of Water-Absorbent Sheet Composition >

A water-absorbent sheet composition cut into a rectangular strip of 8 × 20 cm was used as a sample.

As test solutions, the following Test Solutions A and B were used. Test Solution A (Low-Viscosity Test Solution): Physiological saline (aqueous 0.9% by mass sodium chloride solution) was colored with a small amount of Blue No. 1.

Test Solution B (High-Viscosity Test Solution): In a 10 L vessel were placed 60 g of sodium chloride, 24 g of (anhydrous) sodium carbonate, 60 g of glycerin, and a proper amount of distilled water to completely dissolve. Distilled water was further added to adjust the weight of the overall aqueous solution to 6000 g. Thereafter, the mixed solution was colored with a small amount of Blue No. 1.

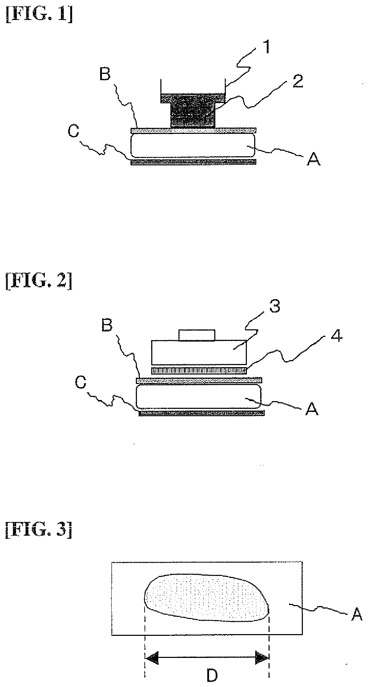

On an upper part of a sample (water-absorbent sheet composition) A was placed a polyethylene air-through style porous liquid-permeable sheet B having the same size as the sample (8 × 20 cm) and a basis weight of 21 g/m$^2$. In addition, underneath the sample was placed a polyethylene liquid-impermeable sheet C having the same size and basis weight as the sheet B, to prepare a body liquid-absorbent article. A cylindrical cylinder 1 having an inner diameter of 2 cm was placed near the central section of this body liquid-absorbent article, and a 20 mL test solution 2 was supplied thereto at one time. At the same time, a time period until the test solution was completely permeated into the body liquid-absorbent article was measured with a stopwatch, which is referred to as a permeation rate (sec) (Figure 1). After 5 minutes, 5 cm × 15 cm filter papers 4, of which mass was previously measured (Wc (g), about 30 g) were stacked near the liquid introductory port, and a 4.5 kg weight 3 having a size of 5 cm × 15 cm was placed thereon (Figure 2). After 5 minutes of applying a load, the mass (Wd (g)) of the filter papers 4 was measured, and an increased mass was defined as the amount of re-wet (g) as follows.

$$\text{Amount of Re-wet (g)} = Wd - Wc$$

Thereafter, the diffusion length D (cm) of a test solution was measured (Figure 3).

[Examples 1 and 2 and Comparative Examples 1 to 3]

A powder mixture prepared by homogeneously mixing a high-water absorbent resin "AQUA KEEP SA60N Type II" (manufactured by Sumitomo Seika Co., Ltd., water retention capacity of saline solution: 40 g/g, water absorption rate of saline solution: 28 s, mass-average particle size: 300 $\mu$m) composed of a partially neutralized product of polyacrylic acid (degree of neutralization 75% by mol) and ethylene-vinyl acetate copolymer (melting temperature: 95°C) used as a hot melt adhesive, in a manner that each of the amounts is as shown as the contents listed in Table 1 based on the sheet area, was evenly spread over a hydrophilic nonwoven fabric (back side) composed of a mixture of rayon fibers and polyethylene terephthalate fibers, having a basis weight of 60 g/m$^2$ (rayon fibers/polyethylene terephthalate fibers = 65/35 (mass ratio)). From the upper part, a hydrophilic nonwoven fabric (front side) composed of a mixture of rayon fibers and polyethylene terephthalate fibers, having a basis weight of 60 g/m$^2$ (rayon fibers/polyethylene terephthalate fibers = 65/35 (mass ratio)) was placed thereon, and the layered nonwoven fabrics were formed into a sheet by means of heat-and-pressure, to give a water-absorbent sheet composition.

[Example 3]

The same procedures as in Example 1 were carried out except that a high-water absorbent resin "AQUA KEEP 10SH-P" (manufactured by Sumitomo Seika Co., Ltd., water retention capacity of saline solution: 41 g/g, water absorption rate of saline solution: 4 s, mass-average particle size: 160 $\mu$m) was used in place of "AQUA KEEP SA60N Type II" in Example 1 to form a sheet, to obtain a water-absorbent sheet composition.

[Comparative Example 4]

The same procedures as in Example 1 were carried out except that a hydrophobic nonwoven fabric (hydrophobic polyethylene terephthalate (100%) having a basis weight of 40g/m$^2$) was used in place of the hydrophilic nonwoven fabric as a nonwoven fabric (back side) to which a water-absorbent resin and a hot melt adhesive were spread in Example 1 to form a sheet, to obtain a water-absorbent sheet composition.

The properties of the water-absorbent sheet compositions obtained in Examples 1 to 3 and Comparative Examples 1 to 4 were measured according to the above methods. The determination results for the presence or absence of liquid leakage upon introduction of a test solution, the permeation rate, the amount of re-wet, and the diffusion length are shown in Table 1.

[Table 1]

| | Kinds of Nonwoven Fabric (Front Side/ Back Side) | Content X of Water-Absorbent Resin [g/m²] | Content Y of Adhesive [g/m²] | Y/X | Thickness [mm] | Liquid Leakage from Body Liquid Absorbent Article | Test Solution A (Low-Viscosity) | | | Test Solution B (High-Viscosity) | | |
|---|---|---|---|---|---|---|---|---|---|---|---|---|
| | | | | | | | Permeation Rate [s] | Amount of Re-wet [g] | Diffusion Length [cm] | Permeation Rate [s] | Amount of Re-wet [g] | Diffusion Length [cm] |
| Ex. 1 | Hydrophilic/ Hydrophilic | 50 | 15 | 0.3 | I 1.0 | Not observed | 11 | 5 | 16 | 79 | 6 | 15 |
| Ex. 2 | Hydrophilic/ Hydrophilic | 100 | 25 | 0.25 | 1.0 | Not observed | 12 | 3 | 13 | 83 | 3 | 13 |
| Ex. 3 | Hydrophilic/ Hydrophilic | 50 | 15 | 0.3 | 1.2 | Not observed | 5 | 8 | 11 | 56 | 7 | 12 |
| Comp. Ex. 1 | Hydrophilic/ Hydrophilic | 5 | 5 | 1.0 | 0.8 | Not observed | 10 | 13 | 20 | 75 | 13 | 20 |
| Comp. Ex. 2 | Hydrophilic/ Hydrophilic | 200 | 50 | 0.8 | 1.0 | Not observed | 16 | 1 | 15 | 113 | 2 | 12 |
| Comp. Ex. 3 | Hydrophilic/ Hydrophilic | 50 | 75 | 1.5 | 1.0 | Not observed | 14 | 16 | 17 | 98 | 16 | 17 |
| Comp. Ex. 4 | Hydrophilic/ Hydrophobic | 50 | 15 | 0.3 | 1.0 | Observed | Undeterminable | | | | | |

It can be seen from the above results that the water-absorbent sheet compositions of Examples 1 to 3 have fast permeation rates, small amount of re-wet regardless of the viscosities of the test solutions, and excellent diffusion of the liquid, as compared to those of Comparative Examples 1 to 3. In addition, in the water-absorbent sheet composition of Comparative Example 4 where a hydrophobic nonwoven fabric was used on the back side, a test solution was not absorbed in a water-absorbent sheet during the introduction of a test solution, and a liquid leakage occurs from the water-absorbent sheet. Therefore, comparative evaluations could not be made.

INDUSTRIAL APPLICABILITY

The water-absorbent sheet composition of the present invention can be used in hygienic material fields, agricultural fields, and construction material fields, among which the water-absorbent sheet composition can be suitably used for sanitary napkins and incontinence pads.

EXPLANATION OF NUMERICAL SYMBOLS

1    cylindrical cylinder
2    test solution
3    weight
4    filter paper
A    sample (water-absorbent sheet composition)
B    liquid-permeable sheet
C    liquid-impermeable sheet
D    diffusion length

**Claims**

1. A water-absorbent sheet composition comprising a structure in which a water-absorbent resin and a hot melt adhesive are sandwiched with two or more sheets of hydrophilic nonwoven fabrics,

   wherein the hydrophilic nonwoven fabrics have a basis weight of 50 to 150 g/m$^2$, and wherein the water-absorbent resin is contained in an amount of from 30 to 100 g/m$^2$,
   wherein the hot melt adhesive is contained in an amount of from 0.20 to 0.5 time that of the amount of the water-absorbent resin contained (based on weight), and
   wherein the water-absorbent sheet composition does not contain hydrophilic fibers between the nonwoven fabrics,
   **characterized in that**
   the water-absorbent resin has a water-retention capacity of physiological saline solution of from 35 to 65 g/g, a water-absorption rate of physiological saline solution of less than 40 s, and a mass-average particle size of 60 to 500 $\mu$m,
   wherein the water-retention capacity, the water-absorption rate, and the mass-average particle size are measured according to the methods described in the description.

2. The water-absorbent sheet composition according to claim 1, wherein the hydrophilic nonwoven fabric is made of at least one member selected from the group consisting of rayon fibers, polyolefin fibers, polyester fibers and mixtures thereof.

3. The water-absorbent sheet composition according to claim 1 or 2, wherein the hot melt adhesive is at least one member selected from ethylene-vinyl acetate copolymer adhesives and stryrenic elastomer adhesives.

**Patentansprüche**

1. Wasserabsorbierende Lagenzusammensetzung, umfassend eine Struktur, in der ein wasserabsorbierendes Harz und ein Schmelzklebstoff zwischen zwei oder mehr Lagen aus hydrophilen Vliesstoffen sandwichartig angeordnet sind,

wobei die hydrophilen Vliesstoffe ein Flächengewicht von 50 bis 150 g/m$^2$ aufweisen, und wobei das wasserabsorbierende Harz in einer Menge von 30 bis 100 g/m$^2$ enthalten ist,
wobei der Schmelzklebstoff in einer Menge enthalten ist, die (bezogen auf das Gewicht) das 0,20- bis 0,5-fache der Menge des enthaltenen wasserabsorbierenden Harzes beträgt, und
wobei die wasserabsorbierende Lagenzusammensetzung keine hydrophilen Fasern zwischen den Vliesstoffen enthält,
**dadurch gekennzeichnet, dass**
das wasserabsorbierende Harz ein Wasserrückhaltevermögen für physiologische Kochsalzlösung von 35 bis 65 g/g, eine Wasserabsorptionsrate für physiologische Kochsalzlösung von weniger als 40 s und eine massengemittelte Partikelgröße von 60 bis 500 μm aufweist,
wobei das Wasserrückhaltevermögen, die Wasserabsorptionsrate und die massengemittelte Partikelgröße gemäß den in der Beschreibung beschriebenen Methoden gemessen werden.

2. Wasserabsorbierende Lagenzusammensetzung gemäß Anspruch 1, wobei der hydrophile Vliesstoff aus mindestens einem Mitglied, ausgewählt aus der Gruppe bestehend aus Rayonfasern, Polyolefinfasern, Polyesterfasern und Mischungen davon, hergestellt ist.

3. Wasserabsorbierende Lagezusammensetzung gemäß Anspruch 1 oder 2, wobei der Schmelzklebstoff mindestens ein Mitglied, ausgewählt aus Ethylen-Vinylacetat-Copolymer-Klebstoffen und Styrol-Elastomer-Klebstoffen, ist.

## Revendications

1. Composition de feuille hydroabsorbante comprenant une structure dans laquelle une résine hydroabsorbante et un adhésif thermofusible sont pris en sandwich entre deux, ou plus, feuilles de tissus non tissés hydrophiles,

dans laquelle les tissus non tissés hydrophiles présentent un poids de base de 50 à 150 g/m$^2$ et dans laquelle la résine hydroabsorbante est contenue en une quantité de 30 à 100 g/m$^2$,
dans laquelle l'adhésif thermofusible est contenu en une quantité de 0,20 à 0,5 fois celle de la résine hydroabsorbante contenue (sur la base du poids) et
dans laquelle la composition de feuille hydroabsorbante ne contient pas de fibres hydrophiles entre les tissus non tissés,
**caractérisée en ce que**

la résine hydroabsorbante présente une capacité de rétention d'eau de solution saline physiologique de 35 à 65 g/g, un taux d'absorption d'eau de solution saline physiologique de moins de 40 s et une taille particulaire moyenne en masse de 60 à 500 μm,
dans laquelle la capacité de rétention d'eau, le taux d'absorption d'eau et la taille particulaire moyenne en masse sont mesurés selon les procédés décrits dans la description.

2. Composition de feuille hydroabsorbante selon la revendication 1, dans laquelle le tissu non tissé hydrophile est composé au moins un élément sélectionné dans le groupe constitué par des fibres de rayonne, des fibres de polyoléfine, des fibres de polyester et des mélanges de celles-ci.

3. Composition de feuille hydroabsorbante selon la revendication 1 ou 2, dans laquelle l'adhésif thermofusible est au moins un élément sélectionné parmi des adhésifs de copolymère éthylène-acétate de vinyle et des adhésifs d'élastomère styrénique.

[FIG. 1]

[FIG. 2]

[FIG. 3]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007219518 A1
- WO 03063746 A1
- US 2006009743 A1
- US 5821179 A
- JP 2004313580 A
- WO 0038749 A1
- GB 2269109 A
- EP 0875225 A1

- JP 2963647 B
- JP 2001252307 A
- JP 6057010 A
- JP 10118114 A
- JP 2000238161 A
- JP 2003011118 A
- JP 2048944 A